# EUROPEAN PATENT APPLICATION

(11) **EP 2 394 641 A1**
(43) Date of publication of application: **14.12.2011**
(21) Application number: 10164416.9
(22) Date of filing: 30.05.2010
(51) Int. Cl.: A61K 9/14, A61K 9/16, A61K 9/20, A61K 31/54

(54) **Pharmaceutical formulations of lornoxicam**

(71) Applicant: Abdi Ibrahim Ilac Sanayi ve Ticaret Anonim Sirketi, Istanbul (TR)
(72) Inventor: Farshi, Farhad, 34555, ISTANBUL (TR); Avci, Recep, 34555, ISTANBUL (TR); Soylemez, Serdar, 34555, ISTANBUL (TR); Parthasarathy, R.G, 34555, ISTANBUL (TR); Baris, Sehriban, 34555, ISTANBUL (TR)

(57) **Abstract**

This invention provides a preparation method of lornoxicam or pharmaceutically acceptable salt thereof characterized in that lornoxicam is dissolved and dissolved lornoxicam is treated onto a pharmaceutical material or mixtures of pharmaceutical materials.

## Description

### Technical Field

The present invention relates to pharmaceutical dosage forms of lornoxicam. This invention is especially describes a particular approach to the lornoxicam and increasing the rapidity of action by being a highly soluble preparation.

### Background Art

Like inappropriate pharmaceutical form of a drug and its administration in an incorrect route, low-solubility properties of an active ingredient may also affect its oral bioavailability or absorption negatively due to insufficient dissolution throughout the gastrointestinal tract. As a matter of fact, more than one-third of the drugs listed in U.S. Pharmacopeia fall into the poorly water soluble or water insoluble categories. Actually, one of the developed strategies for overcoming this solubility problem is to improve the concentration of the drug in solution, which means that regardless of the route of administration, drugs must be in solution to be absorbed. Because of this reason, increasing bioavailability of low-solubility drugs by improving the solubility of them has been one of the greatest challenges to scientist active in pharmaceutical research.

In the art, many efforts have been made to enhance the solubility of drugs in solid dosage forms i.e. solubilization by surfactants or chelating agents, co-milling, crystal modification, pH-control , solid dispersions, prodrug formation and particle size reduction. Among those, although it sometimes may not be translated into good gastrointestinal absorption, particle size reduction method is generally the most widely used one, and there are so many examples comprising this method in the literature. Actually, although there are many methods improved for this purpose, the oral absorption of these molecules is usually limited by solubility.

Most drugs are weak organic acids or bases, existing in un-ionized and ionized forms in an aqueous environment. The proportion of the un-ionized form present is determined by the pH and the drug's pKₐ (acid dissociation constant). The pKₐ is the pH at which concentrations of ionized and un-ionized forms are equal. When the pH is lower than the pKₐ, the un-ionized form of a weak acid predominates, but the ionized form of a weak base predominates. Thus, in plasma (pH 7.4), the ratio of un-ionized to ionized forms for a weak acid (e.g., with a pKₐ of 4.4) is 1:1000; in gastric fluid (pH 1.4), the ratio is reversed (1000:1). Therefore, when a weak acid is given orally, most of the drug in the stomach is un-ionized, favoring diffusion through the gastric mucosa, which affects the bioavailability of it.

Patients suffering from diseases such as acute pain, mild to moderate pain and/or inflammatory conditions and/or related conditions very often require a dosage and a formulation which enable a fast onset of the therapeutic effect of the non-steroidal anti inflammatory drugs (NSAID) substances. As a result, for the drug substances denoted as NSAIDs having weak acidity properties, solubility is a very important issue.

In this invention, Lornoxicam is especially preferred NSAID with low solubility in water and gastric fluid, but other lornoxicam-like NSAID substances may be suitable for to make a composition according to the invention.

Lornoxicam,6-Chloro-4-hydroxy-2-methyl-N-2-pyridinyl-2H-thieno[2,3-e]-1, 2-thiazine-3-carboxamide 1,1-dioxide is a short-acting nonsteroid anti-inflammatory drug (NSAID) from the oxicam group with analgesic, anti-inflammatory and antipyretic properties. It works like other NSAIDs, the inhibitory action on prostaglandin synthesis, via inhibition of cyclo-oxygenase(COX) activity. It is used in the form of injectable, suppository, tablet formulations for relieving postoperative pain following gynaecological or orthopedic surgery, and as effective as other NSAIDs after oral surgery. Lornoxicam was also as effective as other NSAIDs in relieving symptoms of osteoarthritis, rheumatoid arthritis, ankylosing spondylitis, acute sciatica and low back pain.

Like most of the NSAIDs, lornoxicam is very sparingly soluble in water. This insolubility causes serious problem in the formulation of such compounds for oral, parenteral or ophthalmic administration, in particular for intravenous use.

Since lornoxicam is a weak acid (pKₐ of 4.7), the aqueous solubility of acidic lornoxicam is pH dependent. Decreasing pH leads to an increase in the ratio of non-ionized to ionized drug, and in solubility-pH profiles, the solubility of lornoxicam increases exponentially with the increase of pH from 3.0 to 9.0

Fast absorption of drugs into the circulating blood is generally required in managing pain relieves, like in the case of lornoxicam. Therefore, for oral dosage forms it is of utmost importance to have the drug dissolved, completely or partially, already when present in the gastric fluid. Thus, in the event where the drug is not absorbed from the gastric mucosa, it may be ready for being absorbed already when entering the upper intestinal tract, such as duodenum. Duodenum itself has a limited amount of liquid, thus resulting in slow dissolution of the drug in duodenum, although the weak acid may be more soluble in the intestinal fluid. Concordantly, the major problem associated with the formulation and effectiveness of the lornoxicam is its variable oral absorption due to insufficient aqueous solubility at gastrointestinal pH, thus making solubility the rate-determining step in the gastric absorption of it.

PCT/WO 9641646 A (FARMARC NEDERLAND B.V ET.AL.) 27.12.1996 discloses lornoxicam for parenteral and ophthalmic use in the form of a ready-to-use solution or in the form of a product for reconstitution as an aqueous solution. The formulation comprises lornoxicam and a cyclodextrin, or an inclusion complex of it and a cyclodextrin. It also claims use of a buffer to buffer the solution at a pH in the range of from 6,5 to 9 inclusive.

PCT/WO 9532737 A (SOUTH AFRICAN DRUGGISTS LIMITED ET.AL.) 07.12.1995 defines method of making an inclusion complex of cyclodextrin and a sparingly water-soluble NSAID such as lornoxicam, in the form of a paste in which further a wetting solution can be used if necessary.

EP 1109534 B (NYCOMED DANMARK A/S) 12.02.2003 discloses an oral modified release pharmaceutical composition to obtain a relatively fast or quick onset of the therapeutic and/or prophylactic effect. The formulation comprises drug substances (having a pKa value below than 5.5) with a low solubility under acidic conditions and based on a powder that contains the active substance and an alkaline substance which are being contacted under certain conditions. This quick release composition is also based on the particle size distribution of both the powder comprising the active agent and, the powder being contacted with an aqueous medium to form a particulate composition. In that manner, the particles of the particulate composition is below than 180 µm after contact with an aqueous medium.

PCT/WO 9912524 A (NYCOMED DANMARK A/S) 18.03.1999 disclosed modified release multiple-unit formulation of NSAIDs to obtain both a relatively fast or quick onset of the therapeutic effect and the maintenance of a therapeutically active plasma concentration for a relatively long period of time. The formulation comprises two fractions of multiple units; the first fraction quickly releases the drug substance, and the second one which containing sustained release coated multiple units slowly releases the drug substance.

PCT/WO 06000228 A (NYCOMED DANMARK APS) 05.01.2006 defines a manufacturing method of quick release formulation for water insoluble drugs in which the drug substance is not exposed, or at least only to a minimum extent, to any liquid or any aqueous solution during the manufacturing process, and an improved stability is obtained by co-milling of the mixture containing the active drug and alkaline substance. The patent also indicates that a wet granulation method generate stability problems due to the fact that a granulation liquid alters the crystal structure of the active substance.

EP 792147 B (FARMARC NEDERLAND BV) 10.03.2004 identifies inclusion complex of a NSAID and a cyclodextrin, and an alkali agent. In the process, the inclusion complex which is the form of a uniform paste is blended with the alkali agent and with suitable excipients and then formed into a suitable oral dosage forms.

Thus, though many attempts have been made to formulate immediate release formulations of lornoxicam with different manufacturing processes and compositions, these attempts have not targeted design of a method in which particle size distribution of the API was not discussed. As a result, it is invented that quick release formulation of lornoxicam is independent from the particle size of active pharmaceutical ingredient. Namely solubility problem is solved by a method which does not need to adjust particle size of lornoxicam.

There is no prior art which discloses that lornoxicam or pharmaceutically acceptable salts thereof are dissolved and dissolved lornoxicam or pharmaceutically acceptable salts thereof are treated onto the pharmaceutical materials as it is disclosed present invention.

### Summary of invention

The present invention relates to a fast release pharmaceutical composition for oral administration of therapeutically active substances substantially insoluble in water and gastric fluid. Preferably, the present invention provides a quick release formulation for NSAIDs with low solubility in water and gastric fluid.

In another aspect, the present invention provides a quick release oral formulation of lornoxicam via granulation by overcoming the problem of particle size dependency on active drug.

According to the present invention, it has been invented that lornoxicam can be formulated into a quick release forms with reliable absorption, therefore improved tolerability and with dose-proportional bioavailability without employing co-milling or sieving of batches which can be time-consuming and inaccurate by regardless of particle size of lornoxicam or lornoxicam granules .

In another embodiment of the invention is to contact lornoxicam,which is insoluble in water or 0.1 N HCl at room temperature, and alkaline solubility enhancing agent by dissolving in a solvent or mixture of solvents to eliminate the importance of the particle size distribution of the active agent and, of course, extra processes like particle size analysis, sieving, etc. Solvents are, but not limited to water, methanol, ethanol, isopropyl alcohol, acetone, methylene chloride, dichloromethane, mixtures thereof and the like. Said lornoxicam solution may contain further excipient or mixture of excipients. Lornoxicam solution may also contain an inorganic acid or mixtures of inorganic acids such as, but not limited to, formic acid, acetic acid, fumaric acid, tartaric acid, maleic acid, citric acid, succinic acid, malic acid, methanesulfonic acid, p-toluenesulfonic acid and the like.Lornoxicam solution may also contain inorganic acid such as hydrochloric acid solution.

A further embodiment of the present invention is to provide a formulation in which lornoxicam and alkaline solubility enhancing agent are dissolved in a solvent or mixtures of solvents. The preferred solvents are water and ethanol.

In an another embodiment of the invention, there is provided a process for the preparation of a pharmaceutical form of a water insoluble drug suitable for oral administration, said process comprising;
1. dissolving an alkaline substance and with a sufficient quantity of water and alcohol.
2. dissolving lornoxicam in the solution prepared in step a to form granulation solution
3. admixing one or more pharmaceutically acceptable excipients
4. spraying the granulation solution onto said excipient mixture and
5. optionally compressing said mixture into a tablet.

### Technical Problem

In case of low dose compositions, proper content uniformity and release profiles are major concerns in the process development. Proper blending and prevention of segregation are required for a successful processing.

To minimize the segregation potential by improving content uniformity across the granule particle size distribution, thereby improving content uniformity in the tablet.

Particle size adjusting is vital point for lornoxicam or lornoxicam granules. It needs extra processes like particle size analysis, sieving, etc. This problem can be seen from EP 1109534. To eliminate the importance of the particle size distribution of lornoxicam or granules of lornoxicam new methods should be invented.

### Solution to Problem

In order to eliminate the content uniformity problem in case of using greater particles of active pharmaceutical ingredient it is invented that lornoxicam or pharmaceutically acceptable salts thereof are dissolved and treated onto pharmaceutical materials.

This method does also not require adjusting of particle size of lornoxicam or lornoxicam granules. Even though lornoxicam or lornoxicam granules has greater particle size, content uniformity is properly provided. Thus by invented method, there is no need to be taken into account the particle size of lornoxicam or lornoxicam granules.

By invented method of the present application, requirements of extra processes like particle size analysis, sieving etc. are eliminated through dissolving of lornoxicam and dissolved lornoxicam is treated onto a pharmaceutical material.

### Description of embodiments

The present invention relates to a pharmaceutical oral dosage form of lornoxicam by regardless of particle size of lornoxicam or lornoxicam granules.

In another aspect, the present invention provides a pharmaceutical composition comprising lornoxicam , which is substantially insoluble in water and 0.1 N HCl at room temperature, alkaline solubility enhancing agent and pharmaceutically acceptable excipients, characterized in that the pharmaceutical composition has an enhanced solubility without using any particle size adjusting methods. It means that only fewer production steps are needed.

Accordingly a further aspect of the invention that it comprises a pharmaceutical composition in the form of tablet comprising lornoxicam as an active agent having low solubility property, an inorganic salt as an alkaline agent, and one or more fillers, binders, disintegrants, lubricants, mixtures thereof and the like.

In another aspect, the invention provides an oral dosage form with quick release of the active substance in that the oral dosage form comprises the active drug substance as defined herein in contact with one or more alkaline substances.

In another aspect of this invention, lornoxicam or a pharmaceutically acceptable salt thereof is dissolved and dissolved lornoxicam is treated onto a pharmaceutical material or mixtures of pharmaceutical materials. Pharmaceutical material means any kind of forms such as pellets, active or non-active containing tablet cores, excipients, granules, mixtures thereof and the like. Treatment means, but not limited to, adsorbtion or absorbtion or coating or adhering or mixtures thereof and the like.

In this invention, the contact of the active drug with one or more alkaline substances can be obtained by dissolving said active drug and said one or more alkaline substances in a solution, whereby any kind of co-milling process is unnecessary.

The solution may contain water and alcohol mixture the most preferred solvent for dissolving the active substance and the alkaline substance is water-ethanol mixture.

In the present invention, different solvent or solvent mixtures were tried and the best result was obtained by using water-ethanol mixture where the proportion of water to ethanol lies between 4:1-1:4 by weight. In general, if one that is skilled in the art wants to dissolve those in only water, he should use huge amount of it that may result in difficulties in manufacturing process. For example, since the spraying solvent will be prepared in large volume, extra time for spraying will be necessary. Additionally, there may also occur impurity problem during the drying granules, since the time and the heat needed for drying are proportional with the amount of solvent.

In another aspect, the present invention makes it possible to prepare any kind of formulations. Solution of lornoxicam is treated onto any kind of pharmaceutical form such as pellets, active or non-active containing tablet cores, excipients, granules etc. which may then be converted into desired pharmaceutical dosage forms.

The addition of antacid and/or alkaline excipients is a formulation approach to enhance the absorption of lornoxicam after oral administration. Actually, the role of the alkaine substance is to create an alkaline diffusion layer in the gastrointestinal tract by increasing the pH of the medium after dissolution. As a result, the dissoluion of the disintegrating tablet comprising an active agent that is substantially insoluble in water and 0.1 N HCl at room temperature also increases, which mean an enhancement in the absorption of the drug.

Another function of an alkaline substance use in the NSAID formulations is to prevent mucosal damage due to NSAID therapy by reducing acid production associated with the administration of the drug. As a different explanation according to the literature, in the case of bicarbonate salts, the stomach and duodenum are covered by a mucus-bicarbonate barrier, which provides primary defense against the strongly acidic gastric luminal content as well.

A number of alkaline substances are known in the art that is suitable for the purpose of the use. Examples of such alkaline substances are, but not limited to, sodium bicarbonate, Sodium carbonate, magnesium carbonate, calcium carbonate, magnesium oxide, magnesium hydroxide, magnesium trisilicate, aluminum hydroxide, aliminum carbonate, potassium bicarbonate, trometamol, sodium phosphates, mixtures thereof and the like.

The dosage forms of the present invention may take any convenient form. They may be in the form of a solid composition such as tablets, capsules, multi-particulates in sachet or capsule form or any other practical dosage form.

The most preferred pharmaceutical form according to this invention is an oral dosage form, such as a tablet. Accordingly a further aspect of the invention comprises a pharmaceutical composition in the form of tablet comprising an active agent having low solubility property, an inorganic salt as an alkaline agent, and one or more fillers, binders, disintegrants or lubricants.

Tablet excipients are employed in the formulation to enhance the bulk properties of the dosage form and to effect the desired release profiles. So, the tablet comprising lornoxicam according to present invention may combine with an excipient or mixtures of excipients. These excipients typically include diluents or fillers, which add bulk to a formulation to enable formulations of a desired size to be prepared; binders or adhesives, which promote the adhesion of the particles of a formulation to maintain the integrity of the dosage form; disintegrants or disintegrating agents, which promote the break-up of the dosage form after ingestion to make the ingredients more readily available; anti-adherents, glidants or lubricants, which enhance the flow of the tabletting materials, for example into tablet dies, prevent sticking of the formulation to tabletting machine; and miscellaneous adjuvants such as colorants and flavourants.

Suitable diluents include pharmaceutically acceptable inert fillers such as microcrystalline cellulose, lactose, sugar alcohols, calcium phosphates, saccharides, and/or mixtures of any of the foregoing. Examples of diluents include microcrystalline cellulose such as Avicel pH 112, Avicel pH101 and Avicel pH102; lactose such as lactose monohydrate, lactose anhydrous and spray dried lactose ,Calcium phosphate such as Calcium phosphate Dibasic Anhydrous, Calcium phosphate, Dibasic Dihydrate, Tricalcium phosphate ; Sugar alcohol such as mannitol, sorbitol, maltitol etc Saccharides includes fructose; sucrose; glucose or mixtures thereof and the like. Diluents are carefully selected to match the specific formulation with attention paid to the compression properties

Examples of binders are, but are not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylpyrrolidone, gelatin, polyethylene glycol, starch, pre-gelatinized starch, sugars, trehalose, glucose, tragacanth, sorbitol, acacia, alginates, carrageenan, xanthan gum, locust bean gum and gum arabic, waxes, polyacrylamide, mixtures thereof, and whatsoever.

Examples of disintegrants are, but not limited to, sodium starch glycolate, sodium carboxymethyl cellulose, calcium carboxymethyl cellulose, croscarmellose sodium, crospovidone, chitosan, agar, alginic acid, calcium alginate, methyl cellulose, microcrystalline cellulose, powdered cellulose, lower alkyl substituted hydroxypropyl cellulose, hydroxylpropyl starch, low-substituted hydroxypropyl cellulose, polacrilin potassium, starch, pregelatinized starch, sodium alginate, magnesium aluminum silicate, polacrilin potassium, povidone, sodium starch glycolate, mixtures thereof and the like.

Examples of glidants are, but not limited to, silicon dioxide, colloidal silicon dioxide, calcium silicate, magnesium silicate, magnesium trisilicate, talc, starch, mixtures thereof or whatsoever.

Examples of lubricants are, but not limited to, calcium stearate, glyceryl monostearate, glyceryl behenate, glyceryl palmitostearate, hydrogenated vegetable oil, light mineral oil, magnesium stearate, mineral oil, polyethylene glycol, poloxamer, sodium benzoate, sodium lauryl sulfate, sodium stearyl fumarate, stearic acid, talc, zinc stearate, mixtures thereof and the like.

Examples of coloring agents are, but are not limited to, red oxide of iron, yellow oxide of iron, and ready color mix system like Opadry ®.

One or more of these additives can be selected and used by the skilled in the art considering the particular desired properties of solid oral dosage form. The amount of all these additives may vary within the ranges conventional in the art.

The coating formulation may also include additives to promote the desired immediate release characteristics or to ease the application or improve the durability or stability of the coating. Types of additives include plasticizers, pore formers, film formers, anti-tacking agents, opacifiers and glidants. Examples of coating additives suitable for use in the composition of the present invention include plasticizers, such as mineral oils, petrolatum, lanolin alcohols, polyethylene glycol, polypropylene glycol, triethyl citrate, sorbitol and others known in the art. for plasticizers propylene glycol is particularly prepared.

Coating is conducted in conventional fashion, typically by dipping, fluid-bed coating, spray-coating, or pan-coating. The immediate release coating may also be applied using powder coating techniques well known in the art. In these techniques, the drug is blended with optional coating excipients and additives, to form an immediate release coating composition.

The ranges of ingredients by percentage based on total pharmaceutical composition is shown on Table 1 below:

**Table 1**

| **THE RANGES OF INGREDIENTS BASED ON BY WEIGHT OF TOTAL PHARMACEUTICAL COMPOSITION** | |
|---|---|
| **Ingredient** | **Quantity (%)** |
| Active Agent | 1-25% |
| Alkaline agent | 5-40% |
| Filler/Diluent | 10-85% |
| Disintegrant | 5-30% |
| Binder | 1-15% |
| Lubricant | 0.01-2% |
| Film forming agent | 0.5-3% |
| Anti tacking agent | 0.5-5% |
| Plasticizer | 0.01-2% |
| Opacifier | 0.5-5% |

The percentages of ingredients based on by weight of total pharmaceutical composition is shown on Table 2 below:

**Table 2**

| **PERCENTAGES BASED ON BY WEIGHT OF TOTAL PHARMACEUTICAL COMPOSITION** | | |
|---|---|---|
| **Ingredients** | **mg** | **%** |
| Lornoxicam | 8 | 2,4% |
| Alkaline agent | 40 | 12,0% |
| Filler/Diluent | 206,4 | 62,0% |
| Disintegrant | 48 | 14,4% |
| Binder | 16 | 4,8% |
| Lubricant | 1,60 | 0,5% |
| Film forming agent | 5,211 | 1,6% |
| Anti tacking agent | 3,292 | 1,0% |
| Plasticizer | 1,005 | 0,3% |
| Opacifier | 3,292 | 1,0% |
| **Total** | **332,800** | **100,0%** |

The percentages of ingredients based on by weight of tablet core is shown on Table 3 below:

**Table 3**

| **PERCENTAGES BASED ON BY WEIGHT OF TABLET CORE** | | |
|---|---|---|
| **Ingredients** | **mg** | **%** |
| Lornoxicam | 8 | 2,5% |
| Alkaline agent | 40 | 12,5% |
| Filler/Diluent | 206,4 | 64,5% |
| Disintegrant | 48 | 15,0% |
| Binder | 16 | 5,0% |
| Lubricant | 1,60 | 0,5% |
| **Total** | **320** | **100,0%** |

In another aspect, this invention provides a preparation method comprising steps of :
1. Dissolve a portion of an alkaline agent in purified water and then add ethyl alcohol
2. Dissolve the active agent to the step 1
3. Shift filler, remaining of the alkaline agent, disintegrant and binder and then mix
4. Load materials of step 3 in FBP top spray assembly
5. Spray the solution of step 2 over step 4 materials and then dry
6. Sift lubricant through a mesh and then mix with step 7
7. Compress the lubricated blend
8. Disperse film forming agent in purified water. To this add plasticizer, anti tacking agent and opacifier. Homogenize the dispersion
9. Coat the compressed tablets by using the coating dispersion

This invention is also related to in vitro release tests (Table-4). Accordingly, any suitable dissolution method could be used to reach dissolution profiles. In this invention USP Type-2 apparatus is preferred and used. According to the method which was developed during drug development process, the tablets prepared in accordance with the present invention should exhibit the following dissolution profile when tested in a USP Type 2 apparatus, at 50 rpm, and 37° C and in 0,1 N HCl. The results obtained are summarized on table 4. The results show that the immediate release core released at least 85 wt % of the active Lornoxicam during the first five minutes after administration to the in vitro test media and similarity factor (f2 value) is greater than 50.

**Table 4**

| Time (min) | Lornoxicam Release (wt %) |
|---|---|
| 0 | 0 |
| 5 | 85,5 |
| 10 | 96,6 |
| 15 | 98,9 |
| 20 | 99,9 |
| 30 | 100,2 |
| 45 | 100,6 |

## Claims

1. A preparation method of pharmaceutical composition of lornoxicam or pharmaceutically acceptable salts thereof **characterized in that**: a-) lornoxicam or pharmaceutically acceptable salts thereof and optionally an excipient or mixtures of excipients are dissolved in a solvent or mixtures of solvents and b-) said solution of lornoxicam or pharmaceutically acceptable salts thereof are treated onto a pharmaceutical material or mixtures of pharmaceutical materials.

2. According to claim 1, pharmaceutical solution comprising lornoxicam or pharmaceutically acceptable salts thereof and water and ethanol and optionally further pharmaceutical excipient or mixtures of pharmaceutical excipients.

3. According to claim 2 in solution of lornoxicam or pharmaceutically acceptable salts thereof, water and ethanol are in a weight ratio of from 4:1 to 1:4.

4. According to claim 1, pharmaceutical formulation comprising : lornoxicam or pharmaceutically acceptable salts thereof is from 1% to 25%,alkaline agent is from 5% to 40 %, filler/diluents is from 10% to 85% ,disintegrant is from 5% to 30%, binder is from 1% to 15 %, lubricant is from 0.01 % to 2%,film forming agent is from 0.5% to 3%,anti tacking agent is from 0.5% to 5%, plasticizer is from 0.01 % to 2%,opacifier is from 0.5% to 5% by total weight of pharmaceutical composition.

5. According to claim 4,pharmaceutical formulation comprising : lornoxicam or pharmaceutically acceptable salts thereof is 2,4 %,alkaline agent is 12 %, filler/diluents is 62% ,disintegrant is 14,4 %, binder is 4,8 %, lubricant is 0,5%,film forming agent is 1,6 %,anti tacking agent is 1 %, plasticizer is 0,3 % ,opacifier is 1% by total weight of pharmaceutical composition.

6. According to claim 4, pharmaceutical formulation comprising : lornoxicam or pharmaceutically acceptable salts thereof is 2,5 %,alkaline agent is 12,5 %, filler/diluents is 64,5% ,disintegrant is 15 %, binder is 5 %, lubricant is 0,5% by total weight of tablet core.

7. According to claim 2, lornoxicam solution furtherly includes an alkaline agent or mixtures of alkaline agents.

8. According to claim 7, alkaline agent is sodium bicarbonate.

9. According to claim 1, preparation method comrising steps of :a-)dissolving an alkaline substance and with a sufficient quantity of water and alcohol, b-)dissolving lornoxicam in the solution prepared in step a to form granulation solution,c-)admixing one or more pharmaceutically acceptable excipients,d-)spraying the granulation solution onto said excipient mixture and e-) compressing said mixture into a tablet f-)optionally coating of tablet
